# EUROPEAN PATENT SPECIFICATION

(11) **EP 1 932 546 B1**
(45) Date of publication and mention of the grant of the patent: **04.05.2011**
(21) Application number: 06077145.8
(22) Date of filing: 01.12.2006
(51) Int. Cl.: A61L 9/12

(54) **Air deodorizer**
Luftdesodorisierungsmittel
Désodorisant d'ambiance

(43) Date of publication of application: 18.06.2008
(73) Proprietor: The Procter & Gamble Company, Cincinnati, OH 45202 (US)
(72) Inventor: Bulsink, Dirk Jan, 9203 NX Drachten (NL)
(74) Representative: Morelle, Evelyne Charlotte Isabelle

(56) References cited:
- EP-A1- 0 496 460
- FR-A1- 2 556 242
- US-A- 4 726 519
- US-A1- 2005 211 790

## Description

The invention is related to an air deodorizer.

Air deodorizers for spreading a volatile deodorizing fluid are known in the art. Such deodorizers are usually equipped with a fluid holding container and a wick. A ventilating cap is provided to partially close off the container with the wick. The wick is at least partly positioned in the fluid and soaks up the deodorizing fluid. When the wick is sufficiently exposed to air, e.g. through openings in the cap, the deodorant may be spread. During transport, a lid may provided to close of the container with wick in an air and fluid tight manner.

A disadvantage of the known deodorizer is that it is not possible to get an extra boost of a volatile deodorizing fluid if desired.

When a wick is hollow the problem may arise that fluid flows through the hollow part of the wick. For example, when the container is held upside down, fluid may freely flow through the hollow part of the wick in the cap or lid, which is undesirable. Then, after the container is put in a standing position again, some fluid may rest in the cap or lid, for example a side ridge thereof. Then, when the cap or lid is taken off too much deodorizing fluid may be spread and/or spilled, which again is a waste of fluid. EP0496460 concerns a dispenser disclosing a T-shaped wick with a transverse part wrapped around a spray nozzle. The transverse parts increase the damping area of the wick, on the part above the fluid. A massive elongated part is inserted as a plug into a container. The massive part absorbs a substantial amount of fluid.

Therefore, it is one of the goals of the invention to prevent wasting of fluid in an air deodorizer.

This goal and other goals of the invention can be achieved individually or in combination with the invention.

In a first aspect, the invention comprises an air deodorizer provided with a container for being at least partly filled with a deodorizing fluid and a hollow wick, wherein the container, or a part connected thereto, abuts on the wick around said hollow wick, such that leakage between the outer wall of the wick and the container is substantially prevented, wherein the wick is provided with a seal for preventing leakage through the hollow part of the wick, which seal is at least partly arranged within the hollow part of the wick.

By closing the wick with a seal from the inside, something can be pierced through the seal, e.g. a pump, to make contact with the fluid, while when the seal is still intact, fluid will not pass through the hollow part of the wick. Only afterwards, when the pump has been pierced through the hollow part, through the seal, the fluid may pass through the hollow part with the aid of the pump. Also, the seal may be conveniently positioned at a desired level within the wick without having effect on the deodorizing properties, e.g. under the level of the fluid surface. By equipping the wick with a seal, which seal is arranged within the hollow part of the wick, the inside of the wick can be closed off and less fluid is spilled and wasted. The wick may be closed off from the inside without affecting the deodorizing properties of the wick itself since the outside surface of the wick may be in contact with fresh air.

In an embodiment, the seal preferably is substantially plug shaped. In this way, the seal may for example function as a guiding mechanism for guiding through an elongated element. For example, a fluid transport element such as a pump may be conveniently guided through the inside of the seal, for example through an opening element arranged in the bottom, until the end of the fluid transport element reaches below the general fluid surface, e.g. to pump up fluid.

In an embodiment the seal closes around the fluid transport element such that leakage between fluid transport element and the inside of the seal is prevented.

In a further embodiment the seal is at least partly arranged below a fluid surface level of the container, i.e. when the container is (partly) filled with fluid, the seal is preferably arranged below the fluid surface, at least partly, such that the general fluid surface is lifted, at least in a standing position of the deodorizer.

In an embodiment, during the action of passing the fluid transport element through the seal, it may pierce through an opening element that is provided in the seal. Alignment elements may be used for correct alignment of the fluid transport element with respect to the container and/or the opening element.

In a second aspect, the invention comprises a pump for a deodorizer, provided with attachment elements for attaching the pump to a deodorizing fluid container and a elongated element that extends in a fluid transport direction, in attachment with the container extends through the inside of a hollow wick in said container and whereof at least the end is then in contact with deodorizing fluid, wherein the pump comprises an aligning element for aligning the pump with respect to the container and/or simultaneously attaching the pump.

In clarification of the invention, embodiments thereof will be further elucidated with reference to the drawing. In the drawing:
Figure 1A is a sectional front view of a deodorizer wherein a pump is held above a plug;
Figure 1B is a sectional front view of the deodorizer of figure 1A wherein the pump is lowered with respect to figure 1A;
Figure 1C is a sectional front view of the deodorizer of 1A and 1B wherein the pump is lowered with respect to figure 1B and pierces through an opening element of the plug;
Figure 2A is a sectional front view of a deodorizer with a lid, which is held above the container;
Figure 2B is a sectional front view of the deodorizer of figure 2A, wherein the lid closes off the container;
Figure 3 is a sectional front view of a part of the deodorizer, comprising an opening element;
Figure 4 is an exploded view of a deodorizer with a lid;
Figure 5 is a perspective, schematic view of a deodorizer wherein a pump is held above it.

In this description, identical or corresponding parts have identical or corresponding reference numerals. The exemplary embodiments shown should not be construed to be limitative in any manner and serve merely as illustration.

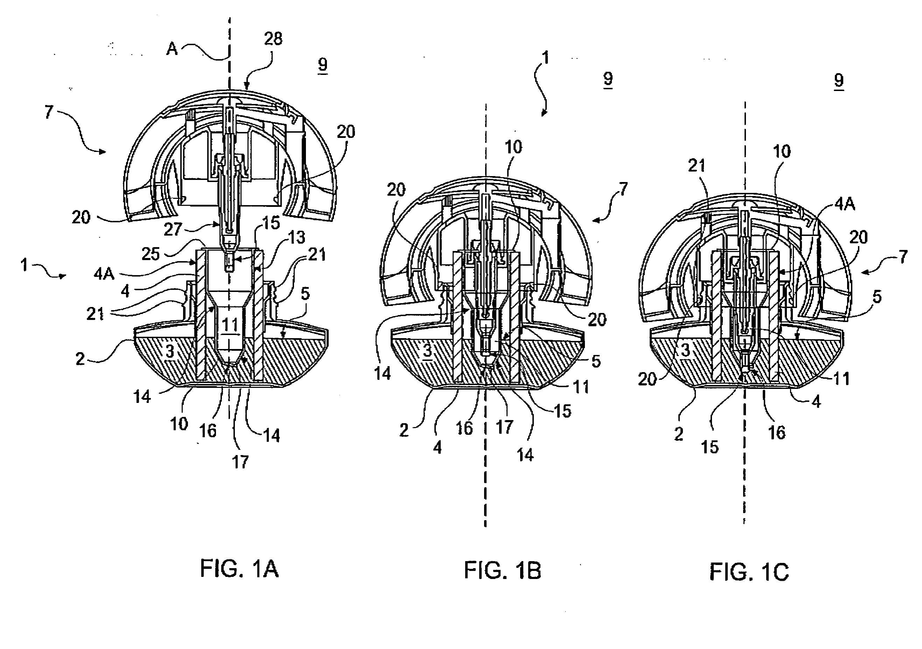

An air deodorizer 1 according to the invention is shown in figures 1A-1C, in a standing position. As can be seen, the deodorizer 1 comprises a container 2 partly filled with a deodorizing fluid 3. A hollow wick 4 is positioned within the container 2, partly under a fluid level 5 such that the wick 4 absorbs fluid 3. When at least an outer surface part 4A of the wick 4 is sufficiently exposed to air, the fluid 3 may be spread in a continuous way by means of the wick 4.

As compared to non-hollow wicks 4, hollow wicks 4 with the same outside dimensions as non-hollow wicks will soak up less fluid 3. Thus, after absorption, the fluid level 5 will not drop as much as compared to non-hollow wicks. The deodorizing properties of a hollow wick 4 may be at least as good or even better as those of a non-hollow wick.

In an embodiment, the wick 4 is equipped with a plug-shaped seal 10, which will be referred to as 'plug' in this description. As can be seen from figures 1A-1C, the plug 10 substantially closes the hollow part 11, i.e. the inside 11, of the wick 4. The plug 10 abuts on the hollow wick 4 such that it seals the inside 11 of the wick 4 at least substantially.

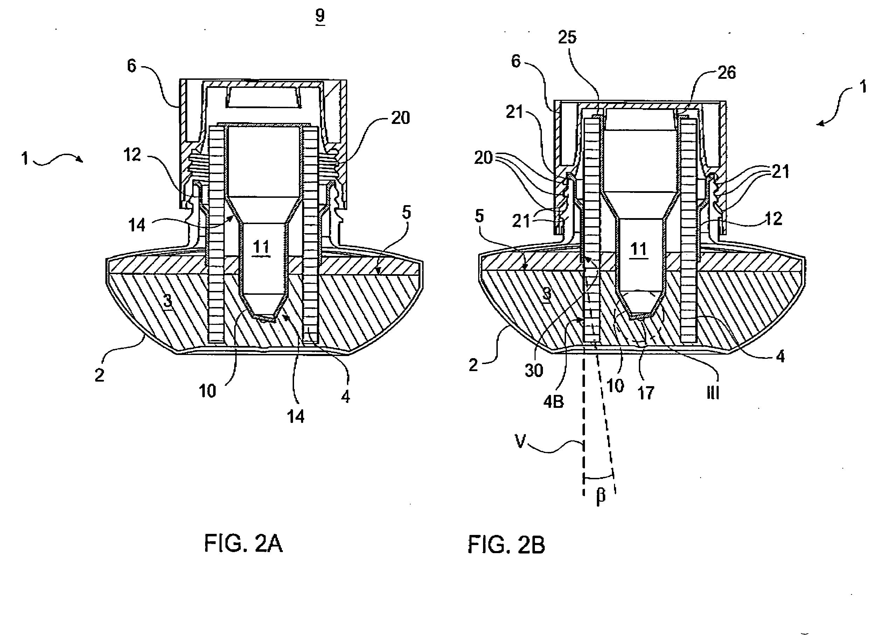

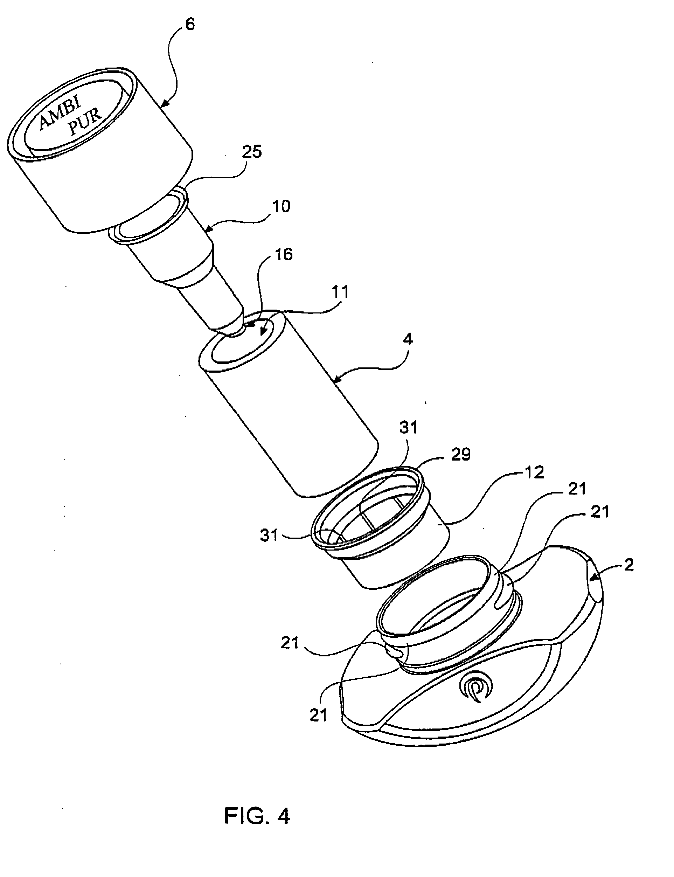

The wick 4 is closed off at its outside by the container 2, or by a wick holder 12 (see figure 2), such that the connection between the wick 4 and the container 2 is substantially sealed, except near vent holes 32 (best indicated in figure 4). These so called vent holes 32 are arranged in the container 2, or in this case, the wick holder 12, so that under pressure in the container 2 is prevented when fluid 3 is absorbed by the wick 4 so that the wick 4 may readily soak up the fluid 3. In principle, almost no fluid 3 will pass by the wick 4 via the inside 11 nor the outside of the wick 4, although, when held upside down some fluid 3 may pass along the vent holes 32, for example into the inside of the lid 6. This fluid 3 will also be allowed to flow back again through the vent holes 32.

In an embodiment, the plug 10 has a partly cylindrical shape. The sealing connection of the plug 10 with the wick 4 can be established by abutment of the plug 10 on at least a part of the inner wall of the wick 4 along a cylindrical outer surface 13. Therefore, the surface 13 of the plug 10 may have approximately the same diameter as the inside surface of the wick 4. Preferably, the dimensions are such, that enough friction is applied between the plug 10 and the wick 4 and the plug 10 stays in position. When for example the deodorizer 1 is put upside down, or inclined, leakage between the plug 10 and the wick 4 may be prevented. The plug 10 may be additionally secured in the wick 4 by a lid 6 (see figure 2A and 2B) or a fluid transport element 8. As can be seen from figure 2B, the lid 6 abuts on the plug 10, near the topside of the plug 10. In this way, the inside of the plug 10 is sealed off.

The plug 10 is provided with a flange 25 that abuts on top of the wick 4. This flange 25 may prevent that the plug 10 is pressed downward through the hollow part 11 of the wick 4. For example, the lid 6, more particularly a flange 26 thereof, and/or the fluid transport element 7 may exert pressure on the plug 10 while the flange 25 of the plug 10 may prevent that the plug 10 is pressed through the inside of the wick 4.

In an embodiment, the plug 10 has such dimensions that the bottom of the plug 10, at least in a standing position of the deodorizer 1, is below the general fluid surface level 5. By putting a part of the plug 10 below the fluid surface level 5, the level 5 is lifted.

In figure 2A and 2B, an embodiment of a deodorizer 1 is shown wherein a lid 6 is provided, with a lid 6 off and lid 6 on, respectively. The lid 6 will prevent early escape of the volatile fluid 2 from the container, for example in storage and/or transport condition. The embodiment shown in figure 1C close to, though not entirely, in a using condition and is provided with a fluid transport element 7. In the using condition, the fluid transport element 7 comprises openings to let deodorant of the volatile fluid 3 pass through, such that eventually fluid 3 will escape from the container 2 more rapidly and/or continuously than in the storage and/or transport condition (e.g. figure 2B).

The fluid transport element 7 may comprise a pump 8 with an actuating element 28, wherein actuation of the element 28 allows for the 'pumping' of extra fluid 3 to the outside 9 of the deodorizer 1, such that the deodorant is spread extra strongly during a particular time period afterwards. The pump 8 may particularly be equipped with an elongated part 27 along a fluid transport direction, that extends through the hollow part of the plug and/or wick 4, for transporting fluid 3 from between the wick 4 to the outside 9. A suction end 15 of the elongated part 27 will be in contact with the deodorizing fluid 3 when the pump is put in the plug 10.

In an embodiment, the plug 10 is substantially hollow and comprises funnel shaped parts 14. The funnel shaped parts 14 may aid in guiding the pump 8 in the plug 10, when the pump 8 is put in the plug 10. Also, the suction end 15 of the pump 8 may be conveniently guided through the centre of the bottom of the plug 10 with the aid of said parts 14. The plug 10 is provided with a flange 25 that abuts on the topside of the wick 4.

Preferably, an opening element 16 is provided near the bottom of the plug 10. The pump 8 is put through the opening element 16 and in contact with the fluid 3, such that fluid 3 can be transported out of the container 2. As shown, the opening element 16 may be positioned below the fluid surface level 5. In this way, the fluid surface level 5 is lifted and just a small part of the pump 8 is put in contact with the fluid 3. After the pump 8 is put through, the opening element 16, or at least the plug 10, seals around the pump 8 such that leaking between pump 8 and plug 10 is prevented at least substantially.

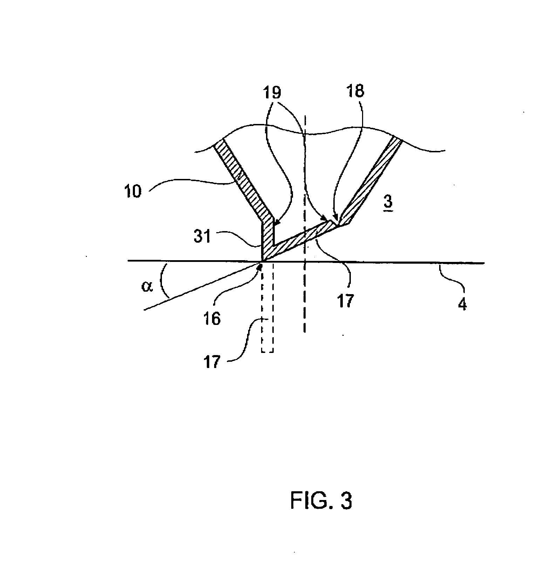

A more detailed view of the opening element 16 is shown in figure 3. As shown, the opening element 16 may comprise a pierce through part 17. The pump 8 may break or push through this part 17 by applying preferably a relatively small amount of force. Preferably the pierce through part 17, or at least the opening element 16, is arranged such that it can be pierced through conveniently. The pierce through part 17 may for example comprise a type of break seal or valve. The part 17 may also be shaped integrally with the plug 10 or be a separate element that is attached to the plug 10 during assembly. In an embodiment the opening element 16 has a cut out 18, at least positioned at the site where the pump 8 will firstly pierce through the pierce through element 17, as can be seen from the drawing. This is advantageous for creating a first opening near the cut out 18 such that less force is needed to have the pump 8 slide through the opening. At an opposite side, the pierce through element 17 may have a tighter connection 31 to the plug 10 such that the pierce through element 17 is not entirely broken off, as indicated in the drawing in dashed lines. For this, the pierce through element 17 may be arranged in an inclined manner, having an inclination with an angle α with respect to a horizontal h, at least in a standing position of the deodorizer 1.

The opening element 16, or at least the plug 10, comprises pump-enclosing parts 19 that enclose the pump 8 such that a substantial sealing connection is made between the pump 8 and the plug 10, even below the fluid surface level 5. For example, when the pump 8, or at least the end 15 thereof, is pierced through the pierce through element 17, no fluid will leak between the pump 8 and the plug 10. Therefore, the opening element 16, particularly the enclosing parts 19 thereof are arranged to close of the pump 8 while it is being pushed through the pierce through element 17. Additionally, the enclosing parts 16 may clamp the pump 8.

In other embodiments, the opening element 10 may for example be configured as a stopper, a valve or another sealing element. In some embodiments fluid 3 and/or air is be allowed to pass between the plug 10 and the pump 8 when the pump is put through the opening element 16.

To connect the fluid transport element 7 to the container 2, it is provided with first attachment elements 20. Also the lid 6 is provided with first attachment elements 20 for connection with the container 2. These first attachment elements 8 aid in establishing a relatively solid connection between the fluid transport element 7 and the container 2 or the lid 6 and the container 2. The container 2 may be provided with second attachment elements 21 to cooperate with the first attachment elements.

In an embodiment, the cooperation of the attachment elements 20, 21 of the fluid transport element 7 and container 2, respectively, comprises a snap-in construction. The first attachment elements 20 may for example comprise protrusions that may be hooked under the second attachment elements 21. As is indicated in steps in figures 1A, 1B and 1C, while pushing through the pump 8, the first attachment elements 20 will be forced towards the outside (figure 1B), in a first step, and will then snap-in to hook under second attachment elements 21 (figure 1C). During the snap-in action, or at least during said pushing of the pump 8, the pump 8 may pierce through the opening element 16. At the same time, a seal between the pump 8 and the plug 10 can be established.

In another embodiment, the fluid transport element 7 is pushed downward until the first attachment elements 20 are located substantially below second attachment elements 21. Then, the fluid transport element 7 is turned around a centre axis A (figure 1A) such that the first attachment elements 20 are positioned under and against the second attachment elements 21. Again, the first attachment elements 20 may be hooked against and/or under the second attachment elements 21 and a suitable attachment is established. During the same action, the fluid transport element 7 may be in correct alignment with the container 2.

The first attachment elements 20 of the lid 6 may for example comprise a screw thread, as indicated in figure 2A. In this way an airtight and/or fluid-tight connection can be established, for example. In an embodiment, the second attachment elements 21 are configured to cooperate with snap-in elements of the fluid transport element 8 as well as with a screw thread of the lid 6, for example.

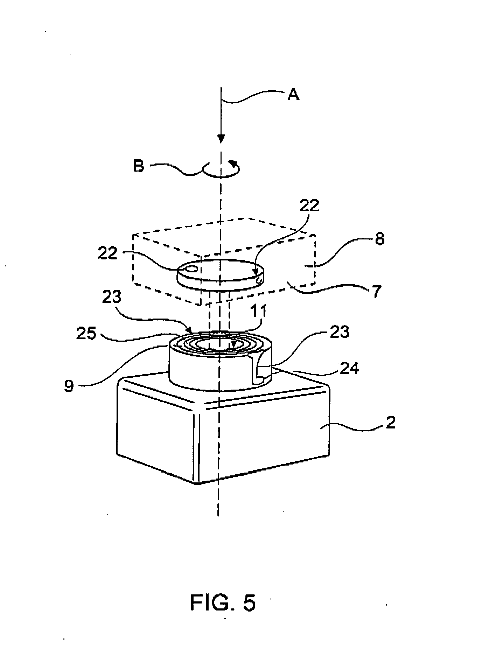

In an embodiment, the fluid transport element 7 comprises alignment elements 22, 23 to align and/or attach the fluid transport element 7 with respect to the container 2. In an embodiment, and for example according to the principle of a bayonet fitting, the fluid transport element 7 is provided with a protrusion 22 and the container 2 with a guide way 23. This is schematically indicated in figure 5. For the sake of clarity, only the part of the fluid transport element 7 that supports the alignment elements 22 is clearly indicated, the rest of the fluid transport element 7 is indicated in dashed lines. To attach the fluid transport element 7 to the container 2 the protrusion 22 is fitted in the guide way 23. Then, the fluid transport element 7 is forced downward in the direction of the axis A while the protrusions 22 are guided along the guide way 23. When the protrusion has approximately reached the lower end of the guide way, the fluid transport element 7 is turned in a direction B around the axis A, until the protrusion 23 abuts on the end 24 of the guide way 23. After releasing the downward force on the fluid transport element 7, a resilient means (not shown) may act between the container 2 and the fluid transport element 7 and exert an upward force so that the protrusion is pushed against the upper side of the end 24 and the fluid transport element 7 is attached to the container 2 relatively securely. With alignment elements 22, 23 the fluid transport element 7 can readily be aligned as well as attached to the container 2, preferably by one movement of the hand. For example, the rotational orientation of the fluid transport element 7 around a centre axis A of the container 2 may be set with the aid of an alignment elements 22, 23. By setting this orientation it can be assured more or less that the pump 8 pierces through the opening element 16 at the side of the cut out 18, such that less force is needed for the piercing through action. Also, the pump 8 can be aligned with respect to the centre of the plug 10. The alignment elements 22, 23 may be conveniently integrated with attachment elements 20, 21.

In an embodiment, the lid 6 comprises an inner flange 26, which abuts on the plug 10, providing for a seal between the plug 10 and the lid 6. When the deodorizer is being transported with the lid 6 on, it could happen that fluid 3 is passed through the absorbing part of the wick 4 into the lid 6, for example when the deodorizer is put in a bottom up or inclined position. For example, when the deodorizer 1 is put back from a bottom up position to a standing up position, the inner flange 26 prevents fluid 3 from flowing into the inside of the plug 10. In a standing position, substantially most or all of the fluid 3 that has passed through e.g. the vent holes 32 will in principle flow back again into the wick 4 or the container 2 via the vent holes 32 and not into the plug 10.

The container 2 can be provided with an additional wick holder 12 that is put in the container 2 for relatively easy fitting of the wick 4 into the container 2. The wick holder 12 is clearly indicated in figure 4. The wick holder 12 may have a flange 29 that abuts on top of the container 2 a corresponding outer diameter to put it in position. With the wick holder 12, the bottom of the wick 4 can for example be held at a distance from the bottom of the container 2 if desired. The wick holder 12 also functions as a seal between the wick 4 and the container 2, i.e. so that less or no fluid will leak between those parts, e.g. in inclined positions of the deodorizer 1. The wick holder 12 may be slightly funnel shaped, at least partly, so that the wick 4 can be put in the container 2 relatively easy while the wick holder 12 clamps it. The funnelled shape is indicated in figure 2B with an angle β, which is the angle between the outside surface 4B of the wick 4, which is approximately a vertical V in a standing position, and the inside surface 30 of the wick holder 12, in a standing position.

The deodorizer 1 can be readily assembled. For example the fluid 3, the fluid transport element 7 and/or the pump 8 can be separate elements that may be assembled, for example by the end user. For example, the pump 8 can be re-used for wherein after or during usage of the deodorizer, less filled or empty containers are replaced by newly filled containers. Also, containers can be switched between for changing fragrances.

Instead of or in addition to a pump 8, in particular embodiments the fluid transport element 7 may for example comprise refilling means, such that a container 2 can be refilled while the hollow wick 4 is in position. Also, the fluid transport element 7 may comprise other elements for emptying the container 2.

Although in the above description mainly plug-shaped seals 10 are described, seals may also be shaped otherwise within the scope of the invention. For example, the seal 10, or a part of it, may be shaped as a thin layer or a foil, preferably in the inside of the wick 4. The seal 10 may abut at least partly on the wick 4 and/or may be connected to and/or integrated with the wick 4. The seal can for example be glued to the inside of the wick 4. Also the seal 10 could be screwed into the wick 4 in some way. The seal 10 or a part of it may comprise a valve. In advantageous embodiments, the seal 10 is arranged such that it closes around the fluid transport element 7 when it is put through the seal 10.

In this description, positional indications such as 'top', 'bottom', 'side' or the like should not be interpreted as being limitative, but are merely used for a proper understanding of the invention. Obviously, these indications refer to a standing position of the deodorizer 1, but are not limitative for other positions.

## Claims

1. Air deodorizer provided with a container (2) for being at least partly filled with a deodorizing fluid (3) and comprising a hollow wick assembly (4) having an annular cross-section, wherein the container (2), or a part connected thereto, abuts on the hollow wick assembly around said hollow wick assembly (4), such that leakage between the outer wall of the wick assembly and the container (2) is prevented, wherein the hollow wick assembly is provided with a seal (10) for preventing leakage through the hollow part (11) of the wick assembly, which seal (10) is at least partly arranged within the hollow part (11) of the wick assembly, **characterised in that** the hollow wick assembly is formed by a wick that is arranged to absorb the fluid (3).

2. Air deodorizer according to claim 1, wherein the seal (10) at least partly extends below a fluid surface level (5) of the container (2), i.e. below the surface of the fluid, when the container (2) is at least partly filled with fluid, at least in a standing position of the deodorizer.

3. Air deodorizer according to claim 2, wherein the seal (10) is plug shaped and hollow.

4. Air deodorizer according to any of the preceding claims, wherein the seal (10) comprises a flange (25) for abutment with the wick on a top surface of the wick, at least in a standing position of the deodorizer.

5. Air deodorizer according to any of the preceding claims, wherein the seal (10) is arranged with an opening element (16) at the bottom.

6. Air deodorizer according to claim 5, wherein a pierce through element (17) is configured for sealing around off a piercing part, such as a pump (8), while it is being pierced.

7. Air deodorizer according to any of the preceding claims, wherein the seal (10) comprises a funnel shaped part (14), arranged for guiding a fluid transport element through the seal (10).

8. Air deodorizer according to any of the preceding claims, further comprising a pump (8), wherein the seal (10) extends through the wick and is hollow, and wherein the fluid transport element extends through the inside of the seal (10), up to a position below the bottom of the seal (10), at least in a standing position of the deodorizer.

9. Air deodorizer according to claim 8, wherein the inside of the seal (10) encloses the fluid transport element to prevent leakage between the fluid transport element and the seal (10).

10. Air deodorizer according to claim 8 or 9, wherein the fluid transport element and the container (2) comprise first and second attachment elements (20, 21), respectively, wherein the first and second attachment elements (20, 21) cooperate for attaching and detaching the fluid transport element to and from the container (2), respectively.

11. Air deodorizer according to any one of claims 8 - 10, wherein the fluid transport element and the seal (10) comprise first and second alignment elements (22, 23), respectively, for aligning the fluid transport element with respect to the seal (10) and/or the container (2).

12. Air deodorizer according to any one of claims 10 - 11, wherein the first alignment element of the fluid transport element comprises a protrusion.

13. Air deodorizer according to any one of claims 10 - 12, wherein the second alignment element comprises a guide way.

14. Air deodorizer according to any one of claims 10 - 13, wherein the alignment elements (22, 23) comprise a bayonet fitting.

15. Air deodorizer according to any of the claims 1 - 8, wherein a lid is provided to close off the container (2) and the wick, wherein in a closed condition the lid has an inner flange (25) that abuts the seal (10), such that fluid is prevented to flow in the hollow part (11) of the wick from the top of the wick.

## Patentansprüche

1. Luft-Desodorierapparat, versehen mit einem Behälter (2), um wenigstens teilweise mit einer Desodorierflüssigkeit (3) gefüllt zu werden, und umfassend eine hohle Dochteinheit (4) mit ringförmigem Querschnitt, wobei der Behälter (2), oder ein daran angeschlossenes Teil, um die hohle Dochteinheit (4) herum so an die hohle Dochteinheit angrenzt, dass eine Leckage zwischen der Außenwand der Dochteinheit und dem Behälter (2) verhindert wird, wobei die hohle Dochteinheit mit einer Dichtung (10) versehen ist, um eine Leckage durch den hohlen Teil (11) der Dochteinheit zu verhindern, wobei die Dichtung (10) wenigstens teilweise in dem hohlen Teil (11) der Dochteinheit angeordnet ist, **dadurch gekennzeichnet, dass** die hohle Dochteinheit aus einem Docht gebildet ist, der so angeordnet ist, dass er die Flüssigkeit (3) absorbiert.

2. Luft-Desodorierapparat nach Anspruch 1, wobei sich wenigstens in stehender Position des Desodorierapparats die Dichtung (10) wenigstens teilweise unterhalb eines Flüssigkeitsniveaus (5) des Behälters (2), d. h. unterhalb der Oberfläche der Flüssigkeit, erstreckt, wenn der Behälter (2) wenigstens teilweise mit Flüssigkeit gefüllt ist.

3. Luft-Desodorierapparat nach Anspruch 2, wobei die Dichtung (10) stopfenförmig und hohl ist.

4. Luft-Desodorierapparat nach einem der vorstehenden Ansprüche, wobei die Dichtung (10) einen hervorstehenden Rand (25) umfasst, der wenigstens in stehender Position des Desodorierapparats an einer oberen Oberfläche des Dochts an dem Docht anliegt.

5. Luft-Desodorierapparat nach einem der vorstehenden Ansprüche, wobei die Dichtung (10) am unteren Ende mit einem Öffnungselement (16) versehen ist.

6. Luft-Desodorierapparat nach Anspruch 5, wobei ein Durchdringungselement (17) so konfiguriert ist, dass es um ein Durchdringungsteil, wie eine Pumpe (8), herum abdichtet, während es durchdrungen wird.

7. Luft-Desodorierapparat nach einem der vorstehenden Ansprüche, wobei die Dichtung (10) einen trichterförmigen Teil (14) umfasst, der zur Führung eines Flüssigkeitstransportelements durch die Dichtung (10) ausgestaltet ist.

8. Luft-Desodorierapparat nach einem der vorstehenden Ansprüche, ferner umfassend eine Pumpe (8), wobei sich die Dichtung (10) durch den Docht erstreckt und hohl ist und wobei sich das Flüssigkeitstransportelement wenigstens in stehender Position des Desodorierapparats durch das Innere der Dichtung (10) bis zu einer Position unterhalb des unteren Endes der Dichtung (10) erstreckt.

9. Luft-Desodorierapparat nach Anspruch 8, wobei die Innenseite der Dichtung (10) das Flüssigkeitstransportelement umschließt, um eine Leckage zwischen dem Flüssigkeitstransportelement und der Dichtung (10) zu verhindern.

10. Luft-Desodorierapparat nach Anspruch 8 oder 9, wobei das Flüssigkeitstransportelement und der Behälter (2) erste bzw. zweite Befestigungselemente (20, 21) umfassen, wobei die ersten und zweiten Befestigungselemente (20, 21) zusammenwirken, um das Flüssigkeitstransportelement an dem Behälter (2) zu befestigen bzw. davon zu trennen.

11. Luft-Desodorierapparat nach einem der Ansprüche 8-10, wobei das Flüssigkeitstransportelement und die Dichtung (10) erste bzw. zweite Ausrichtungselemente (22, 23) umfassen, um das Flüssigkeitstransportelement in Bezug auf die Dichtung (10) und/oder den Behälter (2) auszurichten.

12. Luft-Desodorierapparat nach einem der Ansprüche 10-11, wobei das erste Ausrichtungselement des Flüssigkeitstransportelements einen Vorsprung umfasst.

13. Luft-Desodorierapparat nach einem der Ansprüche 10-12, wobei das zweite Ausrichtungselement eine Führungsbahn umfasst.

14. Luft-Desodorierapparat nach einem der Ansprüche 10-13, wobei die Ausrichtungselemente (22, 23) einen Bajonettverschluss umfassen.

15. Luft-Desodorierapparat nach einem der Ansprüche 1-8, wobei ein Deckel bereitgestellt ist, um den Behälter (2) und den Docht zu verschließen, wobei der Deckel in geschlossenem Zustand einen inneren hervorstehenden Rand (25) aufweist, der an der Dichtung (10) anliegt, so dass verhindert wird, dass Flüssigkeit vom oberen Ende des Dochts in den hohlen Teil (11) des Dochts fließt.

## Revendications

1. Désodorisant d'ambiance pourvu d'un récipient (2) destiné à être au moins partiellement rempli avec un fluide désodorisant (3) et comprenant un ensemble de mèche creuse (4) ayant une coupe transversale annulaire, dans lequel le récipient (2), ou une partie reliée à celui-ci, vient en butée sur l'ensemble de mèche creuse autour dudit ensemble de mèche creuse (4), de telle sorte qu'on empêche une fuite entre la paroi externe de l'ensemble de mèche et le récipient (2), dans lequel l'ensemble de mèche creuse est pourvu d'un joint (10) pour empêcher une fuite à travers la partie creuse (11) de l'ensemble de mèche, lequel joint (10) est au moins partiellement arrangé au sein de la partie creuse (11) de l'ensemble de mèche, **caractérisé en ce que** l'ensemble de mèche creuse est formé par une mèche qui est arrangée pour absorber le fluide (3).

2. Désodorisant d'ambiance selon la revendication 1, dans lequel le joint (10) s'étend au moins partiellement en dessous d'un niveau de surface de fluide (5) du récipient (2), c'est-à-dire en dessous de la surface du fluide, lorsque le récipient (2) est au moins partiellement rempli avec un fluide, au moins dans une position debout du désodorisant.

3. Désodorisant d'ambiance selon la revendication 2, dans lequel le joint (10) est en forme de bouchon et creux.

4. Désodorisant d'ambiance selon l'une quelconque des revendications précédentes, dans lequel le joint (10) comprend un rebord (25) pour mise en butée avec la mèche sur une surface supérieure de la mèche, au moins dans une position debout du désodorisant.

5. Désodorisant d'ambiance selon l'une quelconque des revendications précédentes, dans lequel le joint (10) est arrangé avec un élément qui s'ouvre (16) en bas.

6. Désodorisant d'ambiance selon la revendication 5, dans lequel un élément à percer (17) est conçu pour un scellage autour d'une partie de perçage, tel qu'une pompe (8), pendant qu'il est percé.

7. Désodorisant d'ambiance selon l'une quelconque des revendications précédentes, dans lequel le joint (10) comprend une partie en forme d'entonnoir (14), arrangée pour guider un élément de transport de fluide à travers le joint (10).

8. Désodorisant d'ambiance selon l'une quelconque des revendications précédentes, comprenant en outre une pompe (8), dans lequel le joint (10) s'étend à travers la mèche et est creux, et dans lequel l'élément de transport de fluide s'étend à travers l'intérieur du joint (10), jusqu'à une position en dessous du bas du joint (10), au moins dans une position debout du désodorisant.

9. Désodorisant d'ambiance selon la revendication 8, dans lequel l'intérieur du joint (10) entoure l'élément de transport de fluide pour empêcher une fuite entre l'élément de transport de fluide et le joint (10).

10. Désodorisant d'ambiance selon la revendication 8 ou 9, dans lequel l'élément de transport de fluide et le récipient (2) comprennent des premier et deuxième éléments d'attachement (20, 21), respectivement, dans lequel les premier et deuxième éléments d'attachement (20, 21) coopèrent pour attacher et détacher l'élément de transport de fluide vers et à partir du récipient (2), respectivement.

11. Désodorisant d'ambiance selon l'une quelconque des revendications 8 à 10, dans lequel l'élément de transport de fluide et le joint (10) comprennent des premier et deuxième éléments d'alignement (22, 23), respectivement, pour aligner l'élément de transport de fluide par rapport au joint (10) et/ou au récipient (2),

12. Désodorisant d'ambiance selon l'une quelconque des revendications 10 à 11, dans lequel le premier élément d'alignement de l'élément de transport de fluide comprend une partie saillante.

13. Désodorisant d'ambiance selon l'une quelconque des revendications 10 à 12, dans lequel le deuxième élément d'alignement comprend une voie de guidage.

14. Désodorisant d'ambiance selon l'une quelconque des revendications 10 à 13, dans lequel les éléments d'alignement (22, 23) comprennent un raccord à baïonnette.

15. Désodorisant d'ambiance selon l'une quelconque des revendications 1 à 8, dans lequel un couvercle est fourni pour fermer le récipient (2) et la mèche, dans lequel dans une condition fermée le couvercle a un rebord interne (25) qui vient en butée contre le joint (10), de telle sorte que l'on empêche un fluide de s'écouler dans la partie creuse (11) de la mèche par le haut de la mèche.
